# EUROPEAN PATENT APPLICATION

(11) **EP 3 949 919 A1**
(43) Date of publication of application: **09.02.2022**
(21) Application number: 20779711.9
(22) Date of filing: 16.03.2020
(51) Int. Cl.: A61F 11/08, G10K 15/00, H04R 1/02

(54) **EARPLUG, SOUND COLLECTING DEVICE, AND SOUND VOLUME MEASUREMENT SYSTEM**

(30) Priority: 26.03.2019 JP 2019058326; 31.07.2019 JP 2019141546
(71) Applicant: Nitto Denko Corporation, Ibaraki-shi, Osaka 567-8680 (JP)
(72) Inventor: HATANAKA, Takezo, Ibaraki-shi, Osaka 567-8680 (JP)
(74) Representative: Hoffmann Eitle
(86) International application number: PCT/JP2020/011427
(87) International publication number: WO 2020/196017

(57) **Abstract**

An earplug includes a tubular member configured to extend in one direction and to be capable of bending relative to the one direction, a first acoustic sensor configured to collect sound via the tubular member, and an earplug type sound insulation member in which the tubular member is inserted. The tubular member is preferably bendable according to a shape of an external auditory meatus. A housing having a first opening being communicated with the tubular member is preferably provided. The first acoustic sensor is preferably provided in the housing and collects sound via the tubular member and the first opening.

## Description

### [TECHNICAL FIELD]

The present invention relates to an earplug, a sound collector, and a volume measurement system.

### [BACKGROUND ART]

Conventionally, a device for determining an auditory seal, in the field, provided by an earplug type ear canal device being inserted into an individual's external auditory meatus has been provided. The device includes an acoustic measuring device that detachably engages with an environmental opening in the sound hole of the earplug type ear canal device. The acoustic measuring device includes a probe microphone and a reference microphone isolated from each other and is connected to a data processing unit. The data processing unit includes a control box and a reference sound source connected to the computer unit. The probe microphone and the reference microphone measure a sound pressure level inside the individual's external auditory meatus and a sound pressure level from an environment in proximity to the ear canal device, respectively.

The ear canal device is stretchable and further includes an injection channel for injecting a solidifiable compound material so that the ear canal device is properly shaped into the external auditory meatus (for example, see Patent Document 1).

### [Citation List]

### [Patent Document]

[Patent Document 1] Japanese Translation of PCT International Application Publication No. 2004-524070

### [Summary of Invention]

### [Technical Problem]

In a conventional device, the acoustic measuring device is removed from the environmental opening in the sound hole of the ear canal device after the shape of the device is adjusted to the shape of the external auditory meatus. The ear canal device, as an earplug, is used with the acoustic measuring device removed.

However, the acoustic measuring device is removed when inserting the ear canal device into the external auditory meatus after adjusting the shape of the device into the shape of the external auditory meatus as the earplug. Therefore, a noise condition cannot be measured while a user is wearing the earplug and consequently the user's ear may be exposed to the noise. The user may suffer from noise-induced hearing loss due to continuous exposure to noise.

In consideration of the above, an object of the present invention is to prevent noise-induced hearing loss and to provide an earplug, a sound collector, and a volume measurement system capable of measuring a noise condition in the external auditory meatus while protecting the ear from noise.

### [Solution to Problem]

To solve the above-mentioned problems, an earplug of the present invention includes a tubular member configured to extend in one direction and to be capable of bending relative to the one direction, a first acoustic sensor configured to collect sound via the tubular member, and an earplug type sound insulation member in which the tubular member is inserted.

### [Advantageous Effects of Invention]

According to the present invention, for the purpose of preventing noise-induced hearing loss, a noise condition in the external auditory meatus can be measured while protecting the ear from noise.

### [Brief Description of Drawings]

[Figure 1] FIG. 1 is a diagram illustrating a volume measuring system 200 according to a first embodiment.
[Figure 2] FIG. 2 is a diagram illustrating an actual configuration of an earplug 100.
[Figure 3] FIG. 3 is a diagram illustrating a sound collector 100A.
[Figure 4] FIG. 4 is a diagram illustrating a state in which the earplug 100 is inserted into an external auditory meatus 10 of an ear model 50.
[Figure 5] FIG. 5 is a diagram illustrating the ear model 50.
[Figure 6] FIG. 6 is a diagram illustrating an earplug 100M1 according to a first modification of the first embodiment.
[Figure 7] FIG. 7 is a diagram illustrating frequency characteristics in an external auditory meatus of the earplug 100M1 and the earplug A.
[Figure 8] FIG. 8 is a diagram illustrating frequency characteristics in an external auditory meatus of the earplug 100 and the earplug A.
[Figure 9] FIG. 9 is a diagram illustrating frequency characteristics in an external auditory meatus of the earplug 100M1 and the earplugs A and B.
[Figure 10] FIG. 10 is a diagram illustrating an earplug 100M2 according to a second modification of the first embodiment.
[Figure 11] FIG. 11 is a diagram illustrating an earplug 100M3 according to a third modification of the first embodiment.
[Figure 12] FIG. 12 is a diagram illustrating an earplug 300 according to a second embodiment.
[Figure 13] FIG. 13 is a diagram illustrating an earplug 300A according to a first modification of the second embodiment.
[Figure 14] FIG. 14 is a diagram illustrating an earplug 300B according to a second modification of the second embodiment.
[Figure 15] FIG. 15 is a diagram illustrating an earplug 300C according to a third modification of the second embodiment.
[Figure 16] FIG. 16 is a diagram illustrating an earplug 400 according to a third embodiment.
[Figure 17] FIG. 17 is a diagram illustrating an earplug 400A according to a first modification of the third embodiment.
[Figure 18] FIG. 18 is a diagram illustrating an earplug 400B according to a second modification of the third embodiment.
[Figure 19] FIG. 19 is a diagram illustrating an earplug 400C according to a third modification of the third embodiment.
[Figure 20] FIG. 20 is a diagram illustrating an earplug 400D according to a fourth modification of the third embodiment.
[Figure 21] FIG. 21 is a diagram illustrating an earplug 400E according to a fifth modification of the third embodiment.
[Figure 22] FIG. 22 is a diagram illustrating an earplug 400F according to a sixth modification of the third embodiment.
[Figure 23] FIG. 23 is a diagram illustrating an earplug 500 according to a fourth embodiment.
[Figure 24] FIG. 24 is a diagram illustrating an earplug 600 according to a fifth embodiment.
[Figure 25] FIG. 25 is a diagram illustrating an earplug 600A according to a modification of the fifth embodiment.
[Figure 26] FIG. 26 is a diagram illustrating an earplug 700 according to a sixth embodiment.
[Figure 27] FIG. 27 is a diagram illustrating an earplug 700A according to a modification of the sixth embodiment.

### [Description of Embodiments]

Hereinafter, embodiments to which an earplug, a sound collector, and a volume measurement system of the present invention are applied will be described.

### <First Embodiment>

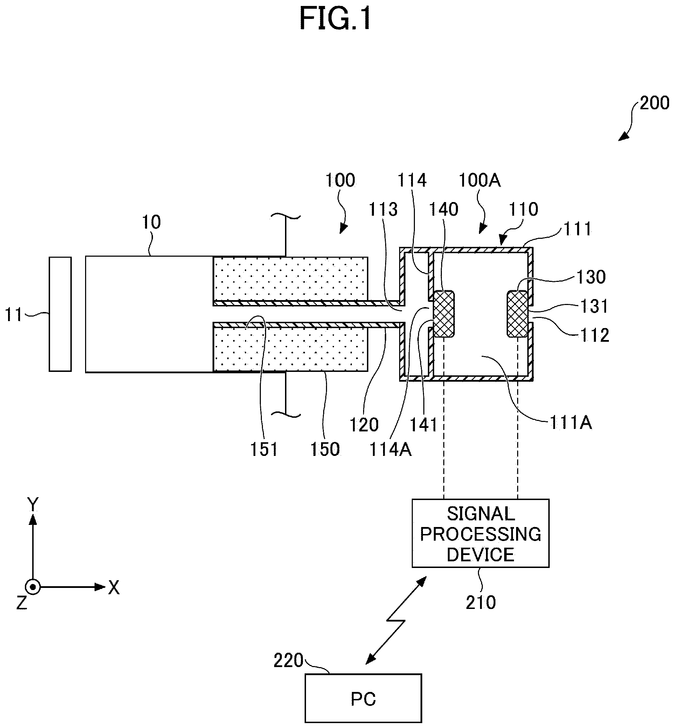

FIG. 1 is a diagram illustrating a volume measuring system 200 according to a first embodiment. The volume measuring system 200 includes an earplug 100 and a signal processing device 210. The following description is provided using the XYZ Cartesian coordinate system.

The earplug 100 includes a housing 110, a tube 120, microphones (hereinafter referred to as a microphone) 130 and 140, and a sound insulation portion 150. The tube 120 is an example of a tubular member, the microphone 130 is an example of a second microphone, the microphone 140 is an example of a first microphone, and the sound insulation portion 150 is an example of a sound insulation member. Further, the earplug 100 excluding the sound insulation portion 150 is referred to as a sound collector 100A.

For the purpose of preventing noise-induced hearing loss, the earplug 100 is a wearable volume measuring device capable of measuring, while protecting an ear from noise (while insulating a sound), the noise condition (frequency, sound pressure, noise time, etc.) outside the external auditory meatus and inside the external auditory meatus, and the difference between these sound pressures.

For example, at a location such as a factory work site and indoor/outdoor construction sites, especially, in a noisy environment where a hand-held tool or a large machine (for example, a press machine) is used to perform an operation, the earplug 100 can measure a noise level near the worker and a noise level at which the worker is actually exposed to noise so that a determination can be made as to whether a noise exposure inside and outside the ear is at a safe level. Also, a sound insulation effect or a wearing state can be determined so that appropriate guidance can be given to a worker who is not wearing the earplug correctly. Further, since the noise level at which the worker is actually exposed to noise can be measured, appropriate working hours can be set and necessary measures and countermeasures can be taken.

Further, the earplug 100 can be used not only at a construction site or the like but also for determining the sound insulation state of the earplug in an environment with a loud noise such as an orchestra or an airport. As noise-induced hearing loss progresses, sound of high frequency range of approximately 2000 Hz to 8000 Hz particularly becomes difficult to be heard.

In general, if an earplug is not worn properly, the earplug cannot exhibit its original sound insulation performance, and the wearer's manager cannot grasp the noise level at which the wearer is actually exposed to noise.

The earplug 100 is provided with microphones 130, 140 which are not removable. If the sound insulation portion 150 is inserted into the external auditory meatus to wear the earplug 100, a determination can be made as to whether the sound insulation portion 150 is appropriately attached to the external auditory meatus to acquire the desired sound insulation performance.

The earplug 100 has the same level of sound insulation as an earplug made of the same sound insulating material as the sound insulation portion 150. The earplug 100 is a device capable of measuring the difference between the volume level inside the external auditory meatus of the wearer and the volume level of the environment in which the wearer is present as the difference in the outputs of the microphones 130 and 140. Hereinafter, each component will be described.

The housing 110 is a case for accommodating two microphones 130 and 140. The housing 110 is made of a material (for example, resin) that is harder than the tube 120. The housing 110 includes a main body 111, openings 112 113, and a partition wall 114. A -X direction side of the housing 110 is directed toward the external auditory meatus 10 and a +X direction side of the housing 110 is directed outward from the external auditory meatus 10.

The main body 111 is a body (main part) of the housing 110, and includes a storage portion 111A for accommodating the microphones 130 and 140 inside. Although the shape of the main body 111 is illustrated in FIG. 1 in a simplified manner, for example, the shape may be a cylindrical member with a central axis parallel to the X-axis, or the cylindrical member with a smaller diameter at both ends in the X-axis direction.

The opening 112 is an example of a second opening, and the opening 113 is an example of a first opening. The opening 112 is provided on the +X direction side of the main body 111 (the side facing outward from the external auditory meatus 10), and the opening 113 is provided on the -X direction side of the main body 111 (the external auditory meatus 10 side).

The partition wall 114 divides the storage portion 111A in the X direction and includes an opening 114A. The opening 114A is aligned with the opening 113 so that the opening 113 is positioned on the extension of the opening.

The tube 120 is attached to the opening 113 of the housing 110 and communicates with the opening 113. The tube 120 is provided to transmit sound inside of the external auditory meatus 10 to the microphone 140. The tube 120 is a tubular member extending in the -X direction from the opening 113, and has a smaller cross section than the cross section of the housing 110 in the YZ plane view.

For example, the tube 120 is made of an elastic member such as silicone which is bendable in the extension direction (X direction). "Bendable" means that it can be bent in a curve so as to have an angle relative to the X direction.

When the sound insulation portion 150 provided on the outer circumference of the tube 120 is pushed to the front of an eardrum 11 inside the external auditory meatus 10, the sound insulation portion 150 deforms according to the shape of the inside of the external auditory meatus 10 and the tube 120 also bends accordingly. In such a state, the tube 120 is configured to be able to maintain a state in which both ends communicate with each other. This is because if the tube 120 is crushed by bending, a state in which both ends are in communication cannot be maintained.

The microphone 130 is provided to collect sound outside the external auditory meatus 10. The sound outside the external auditory meatus 10 is an environmental sound of a subject wearing the earplug 100. The microphone 130 is, for example, a digital microphone with an Analog to Digital (A/D) converter that digitally converts the collected sound to output as a sound signal. An electret condenser microphone or a Micro-Electro-Mechanical Systems (MEMS) microphone can be used as the microphone 130. The MEMS microphone is, for example, a package of a sound pressure sensor formed by the MEMS technology and an Application Specific Integrated Circuit (ASIC) configured to perform signal processing.

The microphone 130 is fixed inside the main body 111 of the housing 110 so as to block the opening 112 at a position where a diaphragm 131 of the microphone 130 substantially coincides with the opening 112. The microphone 130 is connected to a signal processing device 210 by a cable indicated by a dotted line. Therefore, the sound signal representing the sound collected by the microphone 130 is inputted to the signal processing device 210.

The microphone 140 is provided to collect the sound inside the external auditory meatus 10. The microphone 140 includes an A/D converter as an example, and is a digital microphone that digitally converts the collected sound to output as a sound signal. Similar to the microphone 130, the electret condenser microphone or the MEMS microphone can be used as the microphone 140.

The microphone 140 is provided to block the opening 114A inside the main body 111 of the housing 110 at a position where a diaphragm 141 of the microphone 140 substantially coincides with the position of the opening from the +X direction side of the partition wall 114.

The microphone 140 is attached to the partition wall 114 in the housing 110 so as to be apart from the tube 120 without directly contacting the tube 120. Since the tube 120 is the elastic member such as silicone and is relatively soft, vibration may occur in a frequency band of 2000 Hz or higher, for example. If the tube 120 vibrates as described above, the sound in the external auditory meatus 10 cannot be measured correctly by the microphone 140 since the sound caused by the vibration is picked up by the microphone 140.

In consideration of the above, the microphone 140 is attached to the partition wall 114 in the housing 110 so as to be apart from the tube 120 without directly contacting the tube 120 so that the measurement of the microphone 140 is not affected even if the tube 120 vibrates. Further, hardness of the housing 110 to which the tube 120 and the microphone 140 are attached is made harder than the hardness of the tube 120 so that the housing 110 does not vibrate even if the tube 120 vibrates.

The sound insulation portion 150 is an example of a sound insulation member made of a sound insulating material such as a polyurethane sponge. The sound insulation portion 150 is inserted partway in the external auditory meatus 10 so as to seal the space on the inner side of the external auditory meatus 10.

The sound insulation portion 150 includes a through hole 151 positioned on a central axis of a cylindrical sound insulation member. The tube 120 is fixed in the through hole 151 in a state of being inserted. The size of the through hole 151 is matched with the size of the tube 120 so as not to form a gap.

Since the external auditory meatus 10 is actually curved, when the sound insulation portion 150 fixed to the outer circumference of the tube 120 is pushed (or twisted) into the external auditory meatus 10, the sound insulation portion 150 is deformed according to the internal shape of the external auditory meatus 10 and the tube 120 bends accordingly.

The earplug 100 having the configuration described above is made of only a sound insulating material, which is the same material as the sound insulation portion 150, and has the same level of sound insulating as an earplug with the same outer size.

The signal processing device 210 is an example of a volume measuring device. The signal processing device 210 is connected to the output terminals of the microphones 130 and 140, and a sound signal is inputted from the microphones 130 and 140 to the signal processing device 210. The signal processing device 210 includes a processor and a memory. The signal processing device 210 acquires the level difference, by the processor, between the two digital sound signals inputted from the microphones 130 and 140. The data representing the acquired level difference may be stored in the memory.

The signal processing device 210 may be provided inside the storage portion 111A of the housing 110, or may be provided outside the housing 110. For example, if two earplugs 100 are used as a set and the two earplugs 100 are connected by a neck strap (not illustrated), the signal processing device 210 may be provided within the neck strap. The signal processing device 210 is communicably connected to, for example, a Personal Computer (PC) 220 by a wireless communication method.

As the microphones 130 and 140, when using analog microphones that output an analog-format sound signal for representing the collected sound, the signal processing device 210 may include an amplifier circuit and the A/D converter to convert into a digital sound signal.

The signal processing device 210 determines whether the earplug 100 is correctly worn in the external auditory meatus 10 by measuring the difference in volume level measured by the microphone 130 and the microphone 140. Specifically, when the volume level difference is equal to or greater than a predetermined value, the device is determined to be worn correctly. The signal processing device 210 transmits the determination result of the wearing state to the PC 220.

The PC 220 displays the wearing state of the earplug 100 on a display unit such as a liquid crystal display based on the determination result received from the signal processing device 210. For example, the PC 220 provides notification of the wearing state by changing the display color depending on whether the earplug 100 is correctly worn or is not correctly worn (when the earplug is worn abnormally). The PC 220 is an example of a wearing state notification device that provides notification of the wearing state of the earplug 100. Instead of the displaying by the PC 220, the wearing state of the earplug 100 may be provided by notification by a voice from a speaker provided in the earplug 100 or by a vibration of a vibrating element such as a vibrator. Further, the wearing state may be provided by notification via an indication of the light emitted from a light emitting element provided in the housing 110 of the earplug 100. For example, the light emitting element is a Light-Emitting Diode (LED).

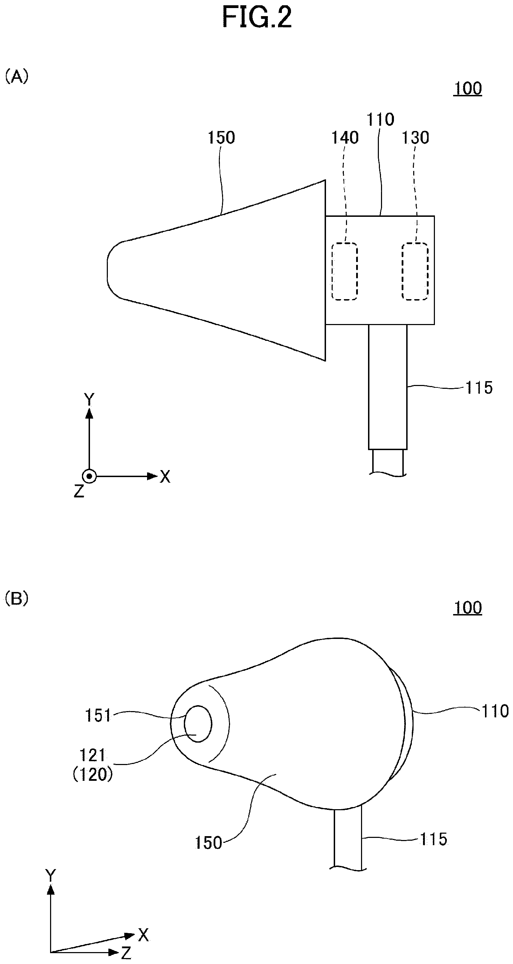

FIG. 2 is a diagram illustrating an actual configuration of the earplug 100 FIG. 2(A) is a side view, and FIG. 2(B) is a perspective view. As illustrated in FIG. 2(A) and FIG. 2(B), the housing 110 is barrel-shaped and contains microphones 130 and 140.

A cable 115 is connected to the housing 110. Sound signals outputted from the microphones 130 and 140 are transmitted to the signal processing device 210 (refer to FIG. 1) by the cable 115. The sound insulation portion 150 has a substantially conical shape, and a tip of the through hole 151 is provided at the end side of the sound insulation portion 150 (end portion in the -X direction) so that the tip of the tube 120 is seen.

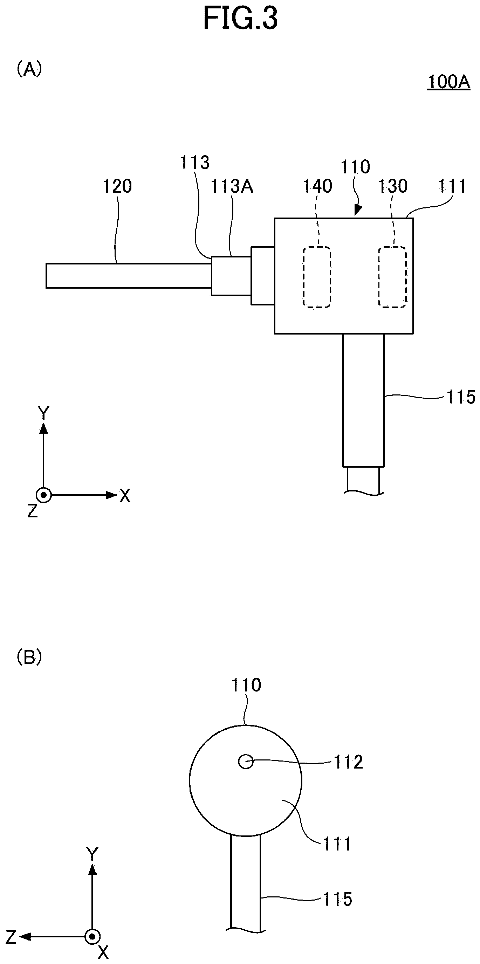

FIG. 3 is a diagram illustrating a sound collector 100A. In FIG. 3, microphones 130 and 140 are omitted. FIG. 3(A) is a side view, and FIG. 3(B) is a rear view (viewed from the +X direction side) .

The housing 110 has a protrusion portion 113A at the end portion on the +X direction side. The protrusion portion 113A is a portion in which a wall portion on the -X direction side of the main body 111 protrudes in a cylindrical shape, and the opening 113 is provided at the tip thereof. The tube 120 may be attachable/detachable to/from the housing 110. If the sound insulation portion 150 and the tube 120 are attachable/detachable to/from the housing 110, for example, the sound insulation portion 150 and the tube 120 can be replaced conveniently when the sound insulation portion 150 and the tube 120 are dirty.

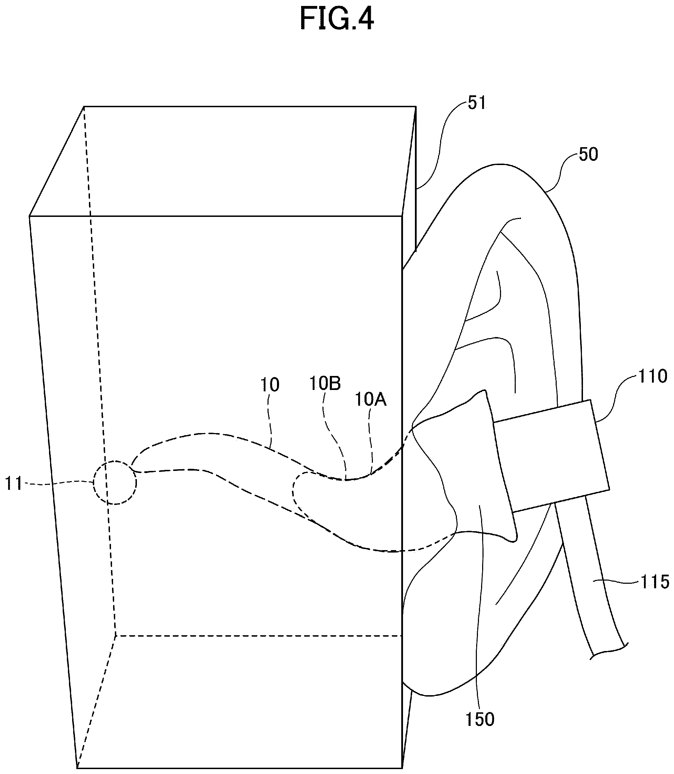

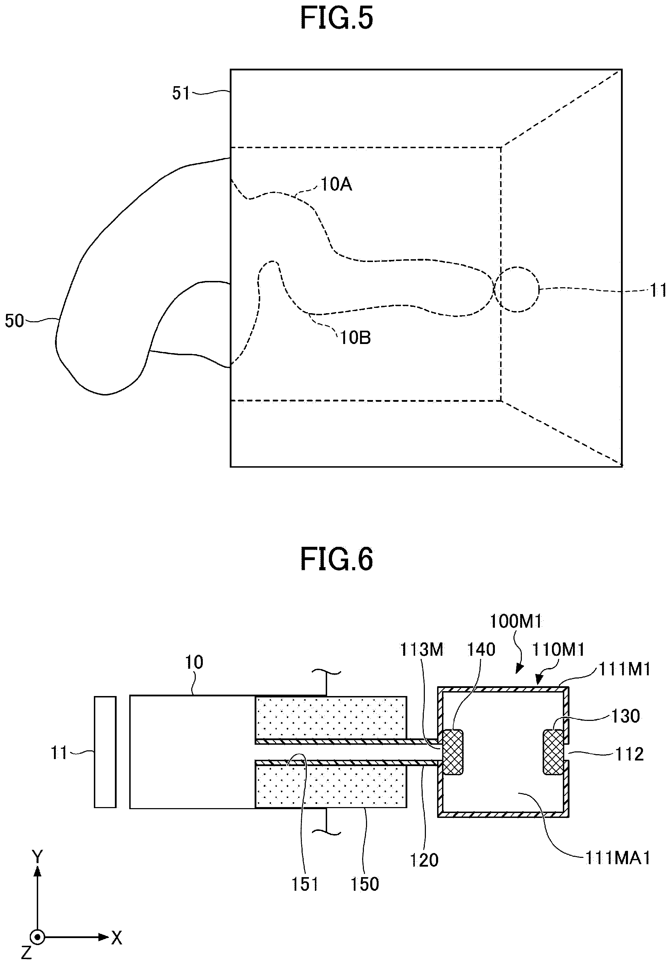

FIG. 4 is a diagram illustrating a state in which the earplug 100 is inserted into the external auditory meatus 10 of an ear model 50. FIG. 5 is a diagram illustrating the ear model 50. The ear model 50 corresponds to the left ear. FIG. 4 illustrates a state in which the left ear is viewed from the front, and FIG. 5 illustrates a state in which the left ear is viewed from above.

The ear model 50 is made of transparent resin and includes the external auditory meatus 10 reproduced to have an average shape of a human external auditory meatus. The average depth of the external auditory meatus 10 is approximately 25 mm to approximately 35 mm from an inlet of the external auditory meatus 10 (opening on a temporal region 51 side) to the front of the eardrum 11.

The external auditory meatus 10 includes, as going inner side from the temporal region 51 toward the eardrum 11, a first curve 10A and a second curve 10B. As described above, the external auditory meatus 10 generally includes two curves (curved portions). The first curve 10A is a portion where the external auditory meatus 10 first curves from the inlet side (the temporal region 51 side) of the external auditory meatus 10 to the inner side. Further, the second curve 10B is a portion where the external auditory meatus 10 curves on the inner side of the first curve 10A, and is a portion in very close proximity to the eardrum 11.

Therefore, in order to obtain a volume level in the external auditory meatus 10 with the earplug 100, the earplug 100 is preferably pushed to be in proximity to the eardrum 11 as much as possible. To implement this, the tube 120 and the sound insulation portion 150 are pushed in until the end of the tube 120 is positioned somewhere along the first curve 10A, at end of the first curve 10A (the exit of the first curve 10A), or are pushed in until the end of the tube 120 is positioned in proximity to the second curve 10B.

In order to position the end of the tube 120 somewhere along the first curve 10A or around the exit of the curve 10A as described above, the tube 120 is configured to be bendable according to the bending of the first curve 10A.

The sound insulation portion 150 is sufficiently softer than the tube 120 and capable of being deformed according to the bending of the first curve 10A of the external auditory meatus 10. Therefore, when the sound insulation portion 150 pushes (or twists) the tube 120 attached to the outer peripheral portion into the external auditory meatus 10, the sound insulation portion 150 is crushed between the inner wall of the external auditory meatus 10 and the tube 120. Then the tube 120 receives a reaction force from the inner wall of the external auditory meatus 10 via the crushed sound insulation portion 150, and bends in accordance with the bending of the first curve 10A.

Therefore, the tube 120 to which the sound insulation portion 150 is attached to the outer peripheral portion can be easily inserted according to the shape of the individual external auditory meatus 10. Further, whether the desired sound insulation performance is acquired in the inserted state can be measured (or determined).

Therefore, it is possible to provide the earplug 100, the sound collector 100A, and the volume measuring system 200 capable of grasping whether they are properly worn in the external auditory meatus 10 while in use. The term "while in use" means the state in which the earplug 100 is worn, and the tube 120 with the sound insulation portion 150 being attached to the outer peripheral portion of the tube 120 is worn (or inserted) in the external auditory meatus 10. That is, it is possible to provide the earplug 100, the sound collector 100A, and the volume measuring system 200 capable of grasping whether they are properly worn in the external auditory meatus 10.

If the earplug-type earplug 100 is not worn in the external auditory meatus 10 correctly, the difference in volume level measured by the microphone 130 and the microphone 140 becomes small since the volume measured by the microphone 140 becomes large. "Wearing the earplug 100 in the external auditory meatus 10 correctly " means that the earplug 100 is properly worn in the external auditory meatus 10 and that the sound insulation portion 150 fits the external auditory meatus 10 so as to appropriately insulate sound.

By measuring the volume level difference with the earplug 100, a quantitative determination can be made as to whether the wearer correctly wears the earplug 100 in the external auditory meatus 10.

In particular, if the earplug 100 is worn in an environment where noise fluctuates or in an environment where loud noise is generated, a determination can be made as to whether sound insulation is properly performed, and this is very effective.

Further, compared to a noise measuring device by fixed point measurement such as being installed in a specific place, by measuring the noise that the wearer is actually exposed to, as in the present invention, the noise level at which the worker, performing the work, is actually exposed to noise can be measured accurately, even in an environment where the workplace changes over time or where the noise level of the work changes over time at the same workplace.

Further, for example, while the worker at the construction site is working with the earplug 100 worn, the data representing the volume level difference may be continuously measured and stored in the memory or the like so that the site manager manages whether the earplug 100 is properly insulating the noise. Further, while the worker at construction site is working with the earplug 100 worn, the data representing the volume level difference may be continuously measured and monitored by the site manager with the PC or the like. Further, while the worker at construction site is working with the earplug 100 worn, the earplug 100 may be equipped with a wireless communication device so that the manager or the worker can check the volume level difference in real time.

Further, the earplug 100 is not required to be made to fit the shape of the individual the external auditory meatus 10 since the tube 120 to which the sound insulation portion 150 is attached bends according to the bending shape inside the external auditory meatus 10. That is, the earplug 100 is suitable for mass production and can be provided at a low cost.

In the above, the configuration in which the microphone 140 is attached to the partition wall 114 in the housing 110 so as to be apart from the tube 120 without directly contacting the tube 120 has been described. However, the microphone 140 may be in direct contact with the tube 120 if the tube 120 does not vibrate or does not affect the measurement of the microphone 140.

### <First Modification of the First Embodiment>

FIG. 6 is a diagram illustrating an earplug 100M1 according to a first modification of the first embodiment. The earplug 100M1 is acquired by replacing the housing 110 of the earplug 100 illustrated in FIG. 1 with the housing 110M1. The housing 110M1 includes a main body 111M1, and openings 112M and 113M. The main body 111M1 does not include the partition wall 114 illustrated in FIG. 1, but includes openings 112M and 113M corresponding respectively to the openings 112 and 113 illustrated in FIG. 1. The microphones 130 and 140 are provided inside the storage portion 111MA1 of the main body 111M1. The diaphragms 131 and 141 of the microphones 130 and 140 are attached so as to be in proximity to the openings 112M and 113M at positions that substantially coincide with the positions of the openings. Therefore, the microphone 140 is in direct contact with the tube 120 attached to the opening 113M.

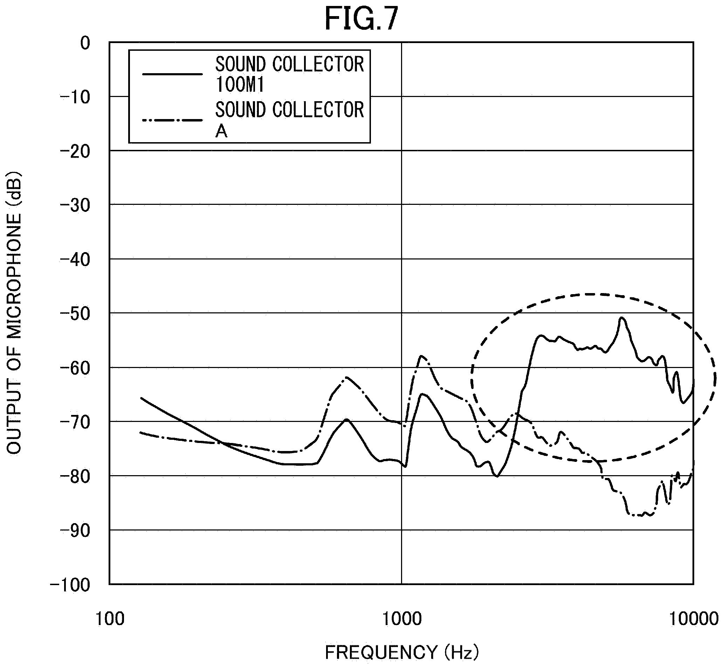

FIG. 7 is a diagram illustrating frequency characteristics in an external auditory meatus. In FIG. 7, the horizontal axis is the frequency and the vertical axis is an output of the microphone 140. Herein, in addition to the earplug 100M1 of the modification, without the tube 120, the output of the microphone 140 measured by the earplug A including the microphone 140 attached to the tip of the sound insulation portion 150 without the through hole 151 is illustrated. A model such as the ear model 50 (refer to FIG. 4) is used for the measurement.

As illustrated in FIG. 7, the earplug A indicated by the alternated long and short dash line is in the range of approximately -58 dB to approximately -87 dB over the entire frequency band from 100 Hz to 10000 Hz.

In the earplug 100M1 indicated by the solid line, the output of the microphone 140 is increased in the high frequency range of approximately 2000 Hz or higher (refer to the part surrounded by the dashed ellipse), and the measurement of the microphone 140 is considered to be affected by the vibration of the silicone tube 120.

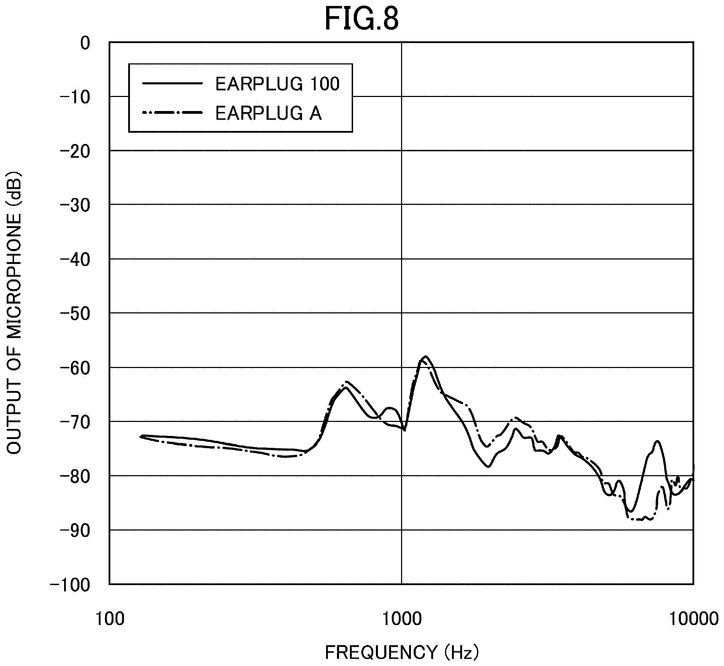

Next, frequency characteristics in the external auditory meatus 10 of the earplug 100 and the earplug A will be described. FIG. 8 is a diagram illustrating the frequency characteristics in the external auditory meatus of the earplug 100 and the earplug A. In FIG. 8, the horizontal axis is the frequency and the vertical axis is the output of the microphone 140. A model such as the ear model 50 (refer to FIG. 4) is used for the measurement.

As illustrated in FIG. 8, the earplug A indicated by the long dash-dot line is in the range of approximately -58 dB to approximately -87 dB over the entire frequency band from 100 Hz to 10000 Hz. Further, the earplug 100 indicated by the solid line illustrates the frequency characteristic of substantially the same level as that of the earplug A.

From the above, it is confirmed that the earplug 100 can measure the sound signal at the same level as the earplug A in which the microphone 140 is attached to the tip of the sound insulation portion 150 without the through hole 151.

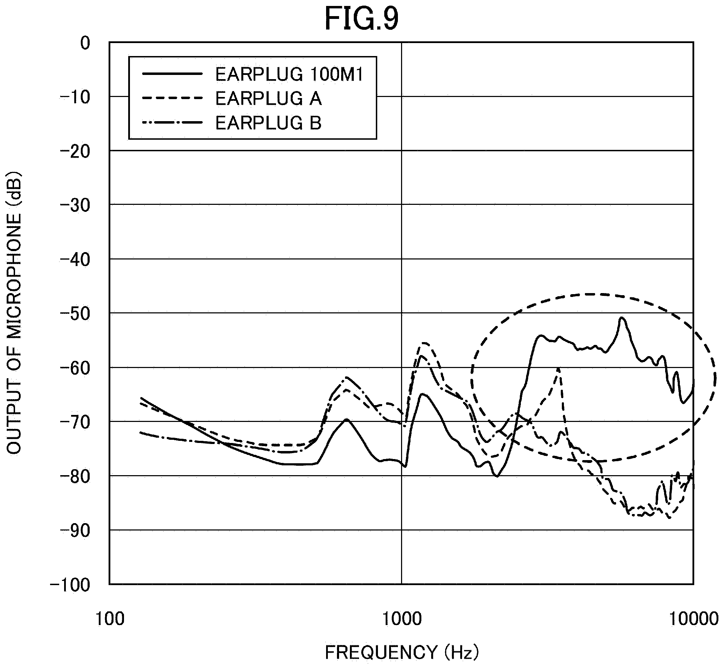

FIG. 9 is a diagram in which the frequency characteristics of an earplug B are added to FIG. 7. In a configuration of the earplug B, the tube 120 of the earplug 100M1 is changed to stainless steel. As illustrated in FIG. 9, although the output of the microphone of the earplug B increases at approximately 3000 Hz, the output of the microphone in the high frequency range is suppressed with respect to the earplug 100M1. This is because since the stainless steel is harder than silicone, the vibration in the high frequency range is considered to have been reduced.

### <Second and Third Modifications of the First Embodiment>

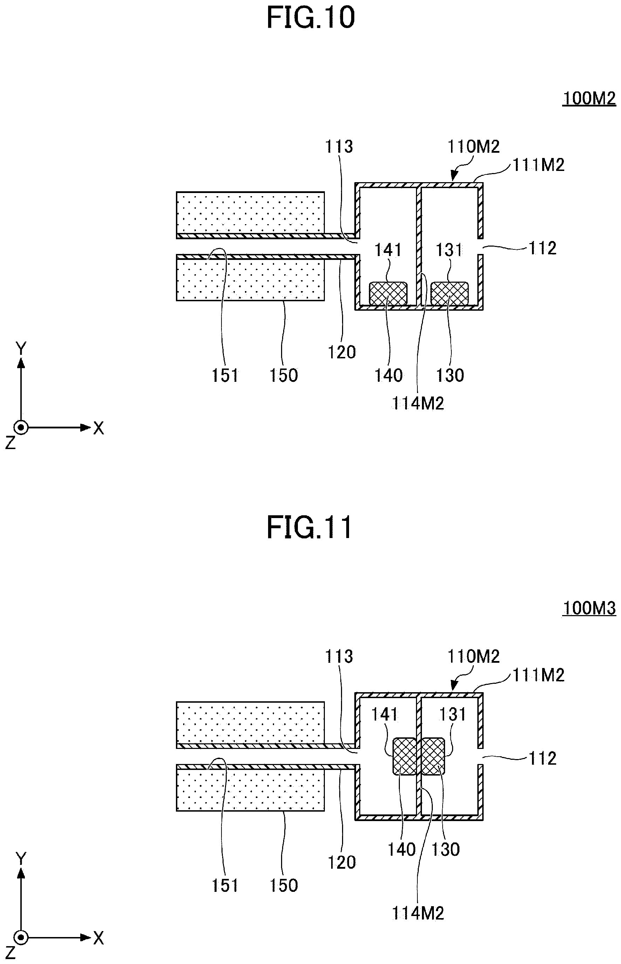

Further, instead of the earplug 100 illustrated in FIG. 1, the earplugs 100M2 and 100M3 illustrated in FIG. 10 and FIG. 11 may be used. FIG. 10 is a diagram illustrating an earplug 100M2 according to a second modification of the first embodiment. FIG. 11 is a diagram illustrating an earplug 100M3 according to a third modification of the first embodiment.

As illustrated in FIG. 10, in the earplug 100M2, the inside of the main body 111M2 of the housing 110M2 is divided into two by a partition wall 114M2. Since the microphone 140 is required only to be apart from the tube 120, the microphones 130 and 140 are provided on the inner wall surface of the housing 110M2 on both sides of the partition wall 114M2. In this case, the diaphragms 131 and 141 of the microphones 130 and 140 are facing upward so that the sound from the openings 112 and 113 can be collected.

In the earplug 100M2 illustrated in FIG. 11, the positions of the microphones 130 and 140 in FIG. 10 are changed to both sides of the partition wall 114M2. The diaphragms 131 and 141 of the microphones 130 and 140 are facing the openings 112 and 113 so that sound can be collected.

### <Second Embodiment>

The earplug 100 according to the first embodiment includes the microphone 130 for collecting sounds outside the external auditory meatus 10 and the microphone 140 for collecting sounds inside the external auditory meatus 10. An earplug according to the present invention is not limited to the configuration having two microphones, and may have only one microphone. Hereinafter, as a second embodiment, an earplug having only one microphone 140 for collecting the sound inside the external auditory meatus 10 as a microphone will be described.

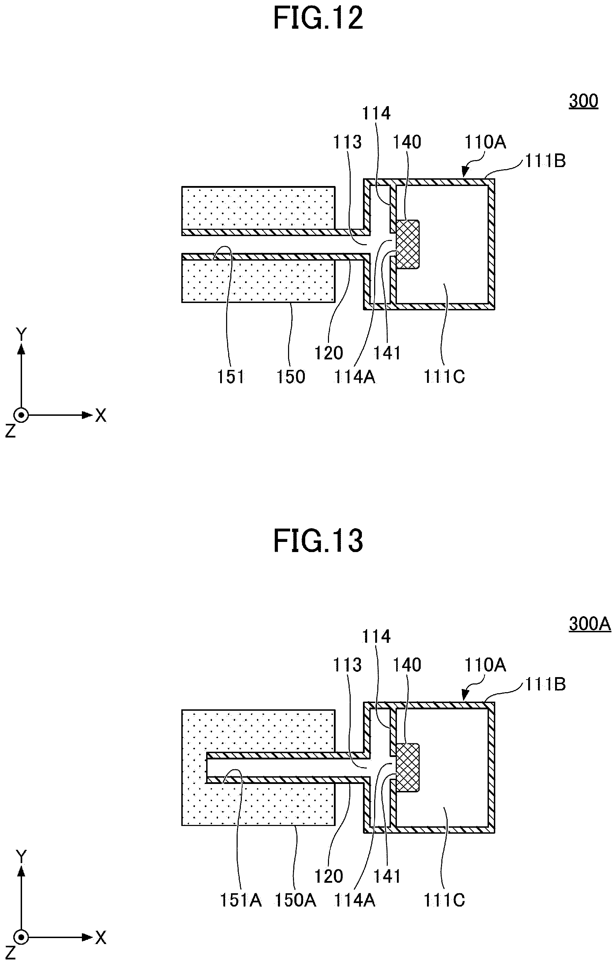

FIG. 12 is a diagram illustrating an earplug 300 according to the second embodiment. In the present embodiment, a housing 110A includes a main body 111B, an opening 113, and a partition wall 114. The main body 111B of the present embodiment is different from the housing 110 of the first embodiment in that only the opening 113 as the first opening is provided without the opening 112 as the second opening. Other configurations of the housing 110A of the second embodiment are the same as those of the housing 110 of the first embodiment.

In the present embodiment, only the microphone 140 for collecting the sound inside the external auditory meatus 10 is provided in a storage portion 111C in the main body 111B. Similar to the first embodiment, the microphone 140 is provided via the diaphragm 141 on the +X direction side of the opening 114A so as to block the opening 114A formed in the partition wall 114.

The tube 120 having the same configuration as that of the first embodiment is connected to the opening 113 of the housing 110A. The sound insulation portion 150 having the same configuration as that of the first embodiment is provided on the outer periphery of the tube 120. The sound insulation portion 150 includes the through hole 151, and the tube 120 is inserted through the through hole 151. Further, the earplug 300 excluding the sound insulation portion 150 corresponds to the sound collector.

In the present embodiment, the microphone 140 is capable of acquiring the volume level inside the external auditory meatus 10. Therefore, with to the earplug 300 according to the present embodiment, the noise condition inside the external auditory meatus can be measured while protecting the ear from the noise for the purpose of preventing noise-induced hearing loss.

Further, in the present embodiment, a signal processing device (not illustrated) quantitatively determines whether the wearer correctly wears the earplug 300 in the external auditory meatus 10 based on the volume level acquired by the microphone 140, because the earplug 300 is not provided with the microphone 130 for collecting sound outside the external auditory meatus 10. For example, the signal processing device determines that the wearer correctly wears the earplug 300 in the external auditory meatus 10 when the volume level acquired by the microphone 140 is equal to or lower than a predetermined value. Further, when an external microphone for collecting external sound is provided separately from the earplug 300, whether the wearer correctly wears the earplugs 300 in the external auditory meatus 10 may be determined quantitatively based on the difference between the volume level acquired by the external microphone and the volume level acquired by the microphone 140.

### <First Modification of the Second Embodiment>

FIG. 13 is a diagram illustrating an earplug 300A according to a first modification of the second embodiment. The earplug 300A according to the first modification has the same configuration as the earplug 300 according to the second embodiment except for a sound insulation portion 150A.

The sound insulation portion 150A of the present modification includes a non-through hole 151A instead of the through hole 151. That is, the sound insulation portion 150A does not penetrate the -X direction side (that is, the eardrum 11 side) and blocks the -X direction side of the non-through hole 151A. The tube 120 of the earplug 300A is inserted into the non-through hole 151A from the +X direction side of the non-through hole 151A. The portion of the sound insulation portion 150A positioned at the tip of the tube 120 performs as an acoustic resistance to sound propagating from the external auditory meatus 10 to the microphone 140.

In the present modification, by providing the non-through hole 151A in the sound insulation portion 150A, the tip of the tube 120 is protected from being exposed from the sound insulation portion 150A. Therefore, when the user wears the earplug 300A, the risk of the tip of the tube 120 being in contact with the eardrum 11 is reduced, and safety is improved. Further, by providing the non-through hole 151A, the inside of the tube 120 and the microphone 140 can be protected (waterproof and dustproof) . The portion of the sound insulation portion 150A positioned at the tip of the tube 120 is formed of a thickness and material sufficient to acquire the sound in the external auditory meatus 10. Although an acoustic signal propagating from the external auditory meatus 10 to the microphone 140 is attenuated by the non-through hole 151A, in signal processing by the signal processing device, the volume level may be determined in consideration of the amount of attenuation of the acoustic signal by the non-through hole 151A.

### <Second Modification of the Second Embodiment>

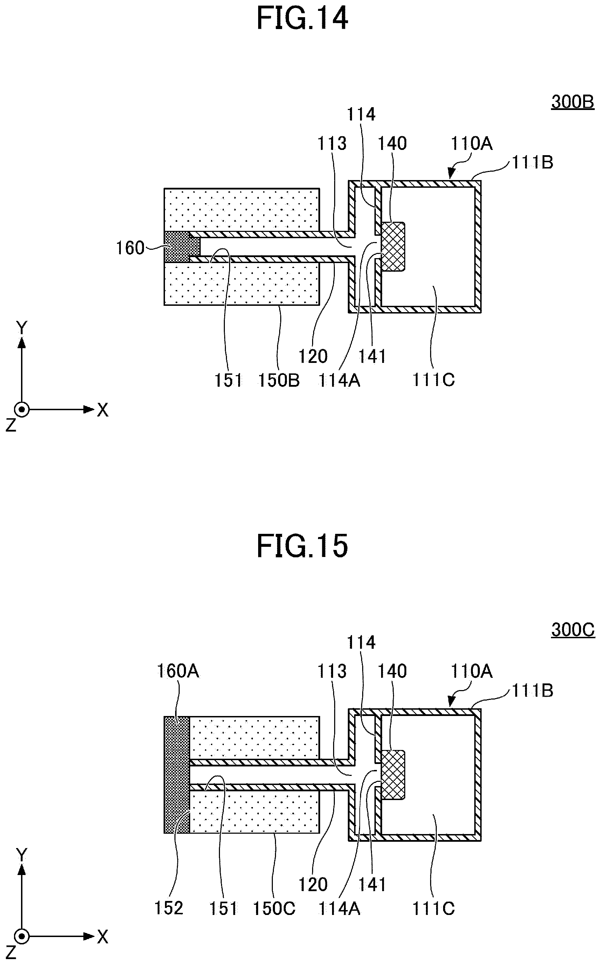

FIG. 14 is a diagram illustrating an earplug 300B according to a second modification of the second embodiment. The earplug 300B according to the second modification has the same configuration as the earplug 300 according to the second embodiment except for a sound insulation portion 150B.

The sound insulation portion 150B of the present modification includes the through hole 151 as in the first embodiment. In the present modification, an acoustic resistor 160 is provided at the tip of the through hole 151 (-X direction side). The acoustic resistor 160 is made of a material having a different property or a different structure from the material forming the sound insulation portion 150B. The acoustic resistor 160 is, for example, an open cell structure such as urethane or polyethylene. The acoustic resistor 160 may be formed of a sound-transmitting film, a waterproof sound-transmitting film, a wire mesh, or the like.

In the present modification, the tube 120 is inserted so that the tip is not exposed from the end (-X direction side) of the through hole 151 and is in contact with the acoustic resistor 160. As illustrated in FIG. 14, in the present modification, a part of the acoustic resistor 160 penetrates into the tube 120, but not limited to this, the acoustic resistor 160 is not required to penetrate into the tube 120.

By providing the acoustic resistor 160 so as to cover the tip of the tube 120 as described above, substantially the same effect as that of the first modification can be obtained.

### <Third Modification of the Second Embodiment>

FIG. 15 is a diagram illustrating an earplug 300C according to a third modification of the second embodiment. The earplug 300C according to the third modification has the same configuration as the earplug 300 according to the second embodiment except for a sound insulation portion 150C.

The sound insulation portion 150C of the present modification includes the through hole 151 as in the first embodiment. In the present modification, an acoustic resistor 160A is formed on the entire end surface 152, which is the end surface of the sound insulation portion 150C on the -X direction side. For example, the acoustic resistor 160A has the same shape as the sound insulation portion 150C in the YZ plane view, and is fixed to the end surface 152 via an adhesive or the like.

The acoustic resistor 160A is made of a material having a different property or a different structure from the material forming the sound insulation portion 150C. The acoustic resistor 160A is, for example, the open cell structure such as urethane or polyethylene. The acoustic resistor 160A may be formed of a sound-transmitting film, a waterproof sound-transmitting film, a wire mesh, or the like.

In the present modification, the tip of the tube 120 is covered with the acoustic resistor 160A. By providing the acoustic resistor 160A so as to cover the tip of the tube 120 as described above, substantially the same effect as that of the first modification can be obtained.

### <Other Modifications>

Further, in the second embodiment and each modification thereof, the microphone 140 is provided in the housing 110A, but the housing 110A is not essential. For example, the housing 110A may be removed. Further, the housing 110A and the partition wall 114 may be removed, and the microphone 140 may be connected directly to the tip (+X direction side) of the tube 120 or may be connected via the diaphragm.

In the second and third modifications of the second embodiment, the acoustic resistor is provided at the tip of the tube 120, but as long as a path from the tip of the tube 120 to the microphone 140 is formed, the acoustic resistor may be provided at any position. For example, the acoustic resistor may be provided in the central portion of the tube 120, the opening 113 of the housing 110A, and the opening 114A of the partition wall 114. Further, the entire inside of the tube 120 may be filled with the acoustic resistor.

Further, the earplug 100 according to the first embodiment may also be provided with the acoustic resistor in the same manner. In this case, the acoustic resistor may be provided in the opening 112 of the housing 110A, in addition to the central portion of the tube 120, the opening 113 of the housing 110, and the opening 114A of the partition wall 114. Further, the entire inside of the tube 120 may be filled with the acoustic resistor.

Further, the configuration of the sound insulation portion illustrated in FIG. 13 to FIG. 15 can be applied to the earplug according to the first embodiment and each modification of the first embodiment.

### <Third Embodiment>

In the earplug according to the first and second embodiments, a microphone for collecting the sound inside the external auditory meatus 10 is provided on the housing side of the tube (the side opposite to the external auditory meatus). Hereinafter, as a third embodiment, an earplug in which a microphone for collecting sound inside the external auditory meatus is provided in the tube will be described.

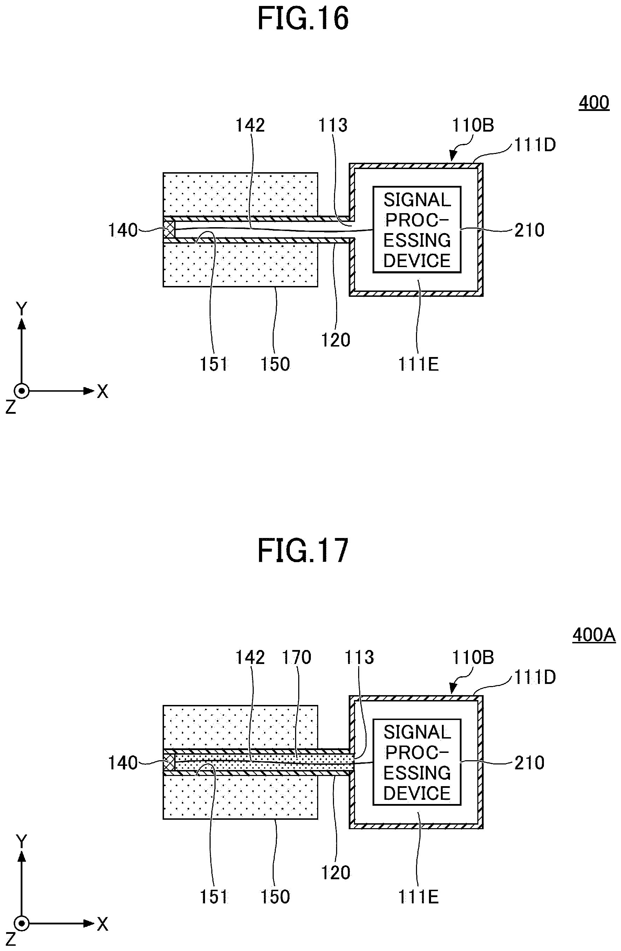

FIG. 16 is a diagram illustrating an earplug 400 according to a third embodiment. In the present embodiment, a housing 110B includes a main body 111D and an opening 113. In the present embodiment, a microphone 140 for collecting sound inside the external auditory meatus 10 is of a size that can be inserted into the tube 120 connected to the opening 113. In the present embodiment, the microphone 140 is arranged in the tube 120 at the end opposite to the opening 113.

In the present embodiment, for example, a signal processing device 210 can be arranged in a storage portion 111E of the housing 110B. The microphone 140 is connected to the signal processing device 210 via a signal cable 142 inserted in the tube 120.

Other configurations of the earplug 400 are the same as the configurations of the earplug 300 according to the second embodiment.

By arranging the microphone 140 at the end of the tube 120 as in the present embodiment, sound collection efficiency of the sound (acoustic signal) inside the external auditory meatus 10 is improved. Further, the transmission of high frequency noise, which may occur due to the vibration of the tube 120, to the microphone 140 is suppressed.

Further, in the present embodiment, since the tube 120 is hollow, the signal cable 142 can be easily fed into the tube 120.

### <First Modification of the Third Embodiment>

FIG. 17 is a diagram illustrating an earplug 400A according to a first modification of the third embodiment. The earplug 400A according to the first modification is filled with a vibration reducing member 170 on a downstream side (the opening 113 side) from the position of the microphone 140 in the tube 120. The earplug 400A has the same configuration as the earplug 400 according to the third embodiment except that the tube 120 is filled with the vibration reducing member 170.

The vibration reducing member 170 is, for example, an open cell structure made of urethane, polyethylene, or the like, to suppress the vibration of the tube 120. The vibration reducing member 170 may be made of the same material as the acoustic resistor 160 illustrated in the second embodiment.

By filling the tube 120 with the vibration reducing member 170 as in the present modification, the transmission of high frequency noise, which may occur due to the vibration of the tube 120, to the microphone 140 can be further suppressed.

In the present modification, the vibration reducing member 170 is filled on the downstream side of the microphone 140 in the tube 120, but the downstream side of the microphone 140 in the tube 120 may be non-hollow (solid).

### <Second Modification of the Third Embodiment>

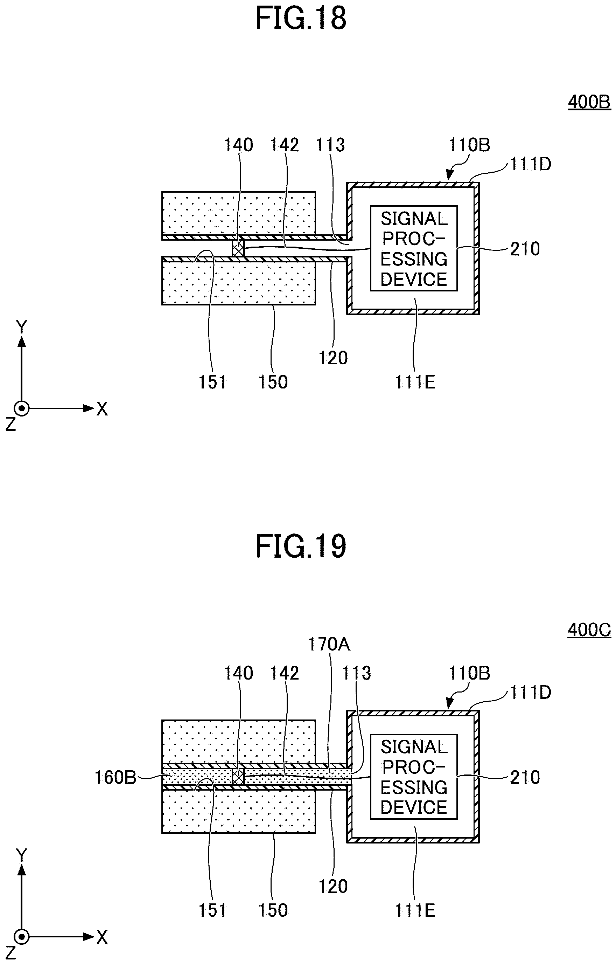

FIG. 18 is a diagram illustrating an earplug 400B according to a second modification of the third embodiment. The earplug 400B according to the second modification is different from the configuration of the earplug 400 according to the third embodiment only in that the microphone 140 is arranged in the central portion in the tube 120.

By arranging the microphone 140 in the central portion of the tube 120 as in the present modification, the microphone 140 can be protected from dirt.

### <Third Modification of the Third Embodiment>

FIG. 19 is a diagram illustrating an earplug 400C according to a third modification of the third embodiment. The earplug 400C according to the third modification is filled with a vibration reducing member 170A on the downstream side (the opening 113 side) from the position of the microphone 140 in the tube 120, and is filled with an acoustic resistor 160B on the upstream side (end side) from the position of the microphone 140 in the tube 120. Other configurations of the earplug 400C are the same as those of the earplug 400B according to the second modification.

The vibration reducing member 170A is made of the same material as the vibration reducing member 170 of the first modification. The vibration reducing member 170A and the acoustic resistor 160B may be made of the same material.

By filling the tube 120 with the vibration reducing member 170A as in the present modification, the transmission of high frequency noise, which may occur due to the vibration of the tube 120, to the microphone 140 can be further suppressed. Further, by filling the side upstream to the microphone 140 in the tube 120 with the acoustic resistor 160B, the microphone 140 can be protected (waterproof or dustproof).

Note that only one of the vibration reducing member 170A and the acoustic resistor 160B may be alternatively provided. Further, the side downstream to the microphone 140 in the tube 120 may be non-hollow (solid).

In the third embodiment and each modification, as in the second embodiment, the microphone 130 for collecting the sound outside the external auditory meatus 10 is not provided, but the microphone 130 may be provided in the storage portion 111E of the housing 110B.

### <Other Modifications>

With respect to the earplug according to the third embodiment and each of the modifications, the sound insulation portion can be deformed in the same manner as in the first to third modified examples of the second embodiment illustrated in FIG. 13 to FIG. 15.

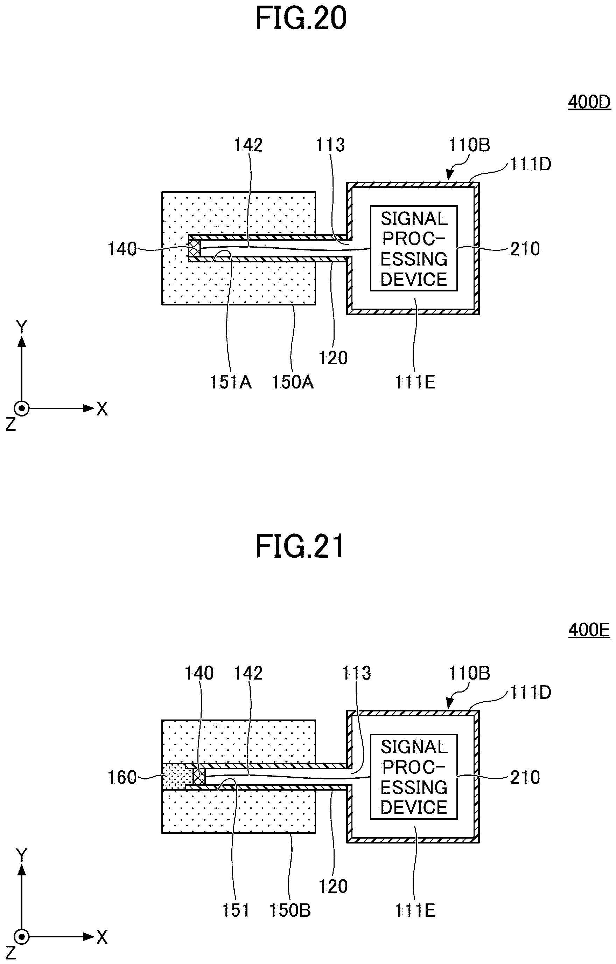

FIG. 20 is a diagram illustrating an earplug 400D according to a fourth modification of the third embodiment. The earplug 400D according to the fourth modification has the same configuration as the earplug 400 according to the third embodiment except for the sound insulation portion 150A. The sound insulation portion 150A has the same configuration as the sound insulation portion 150A illustrated in FIG. 13, and includes a non-through hole 151A instead of the through hole 151.

In the present modification, the microphone 140 arranged at the tip of the tube 120 comes into contact with the sound insulation portion 150A, and the portion of the sound insulation portion 150A that comes into contact with the microphone 140 performs as an acoustic resistance. By providing the non-through hole 151A as described above, the inside of the tube 120 and the microphone 140 can be protected (waterproof and dustproof).

FIG. 21 is a diagram illustrating an earplug 400E according to a fifth modification of the third embodiment. The earplug 400E according to the fifth modification has the same configuration as the earplug 400 according to the third embodiment except for the sound insulation portion 150A. The sound insulation portion 150B has the same configuration as the sound insulation portion 150B illustrated in FIG. 14, and includes the through hole 151.

In the present modification, the acoustic resistor 160 is provided at the tip of the through hole 151 (-X direction side), and the microphone 140 is in contact with the acoustic resistor 160. By providing the acoustic resistor 160, substantially the same effect as that of the fourth modification can be obtained.

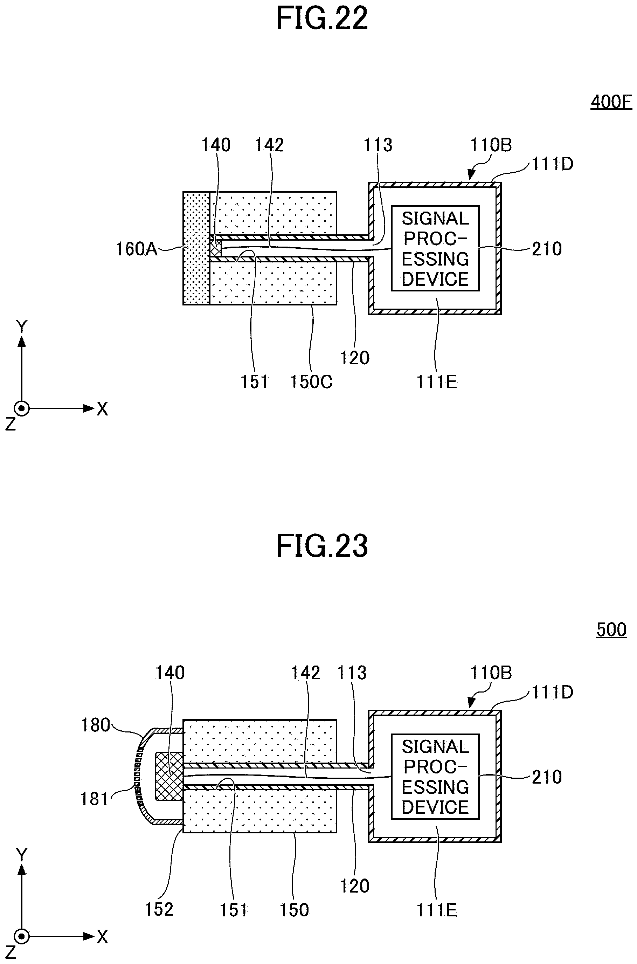

FIG. 22 is a diagram illustrating an earplug 400F according to a sixth modification of the third embodiment. The earplug 400F according to the sixth modification has the same configuration as the earplug 400 according to the third embodiment except for the sound insulation portion 150C. The sound insulation portion 150C has the same configuration as the sound insulation portion 150C illustrated in FIG. 15, and includes the through hole 151.

In the present modification, the acoustic resistor 160A is formed on the entire end surface 152, which is the end surface of the sound insulation portion 150C on the -X direction side, and the microphone 140 is in contact with the acoustic resistor 160A. By providing the acoustic resistor 160A, substantially the same effect as that of the fourth modification can be obtained.

### <Fourth Embodiment>

In the earplug according to the third embodiment, the microphone for collecting the sound inside the external auditory meatus is provided in the tube. Hereinafter, as a fourth embodiment, an earplug in which a microphone for collecting the sound inside the external auditory meatus is attached to the tip of the tube will be described.

FIG. 23 is a diagram illustrating an earplug 500 according to the fourth embodiment. In the earplug 500 according to the fourth embodiment, a microphone 140 for collecting sound inside the external auditory meatus 10 is attached to the tip of the tube 120. The microphone 140 is larger than the inner diameter of the tube 120 and thus is not small enough to be inserted into the tube 120.

In the present embodiment, since the microphone 140 is exposed to the outside of the tube 120, the microphone 140 is covered with a cover 180 attached to the end surface 152 of the sound insulation portion 150. Multiple tiny holes 181 for transmitting sound are formed in the cover 180. The cover 180 is, for example, a mesh-shaped member. The hole 181 is preferably small enough to prevent water and dust from passing through.

By attaching the microphone 140 to the tip of the tube 120 as in the present embodiment, the sound collection efficiency of the sound (acoustic signal) inside the external auditory meatus 10 is improved. Further, the transmission of highfrequency noise, which may occur due to the vibration of the tube 120, to the microphone 140 can be suppressed.

In the present embodiment as well, the tube 120 may be filled with the vibration reducing member, or the tube 120 may be non-hollow (solid). Further, the cover 180 may be filled with the acoustic resistor. Further, the microphone 130 may be provided in the storage portion 111E of the housing 110B.

The housing 110B is provided in the third embodiment, the fourth embodiment, and each modification, but the housing 110B is not essential. Further, the configurations according to the previously described embodiments and the modifications can be combined with each other as long as contradiction does not occur.

Further, the microphones 130 and 140 illustrated in each of the previously described embodiments and the modifications are examples of omnidirectional or unidirectional acoustic sensors. The acoustic sensor includes an electret condenser microphone and MEMS microphone as described previously as well as an acoustic electric converter, a microelectromechanical sensor, an ultrasonic microphone, and the like.

### <Fifth Embodiment>

In an earplug having a microphone 130 for collecting sound outside the wearer in addition to a microphone 140 for collecting sound inside the external auditory meatus 10, as in the first embodiment, the microphone 130 might collect sound of wind (wind noise) outside the earplug. Hereinafter, as a fifth embodiment, an earplug capable of reducing the level of the sound of wind collected by the microphone 130 will be described.

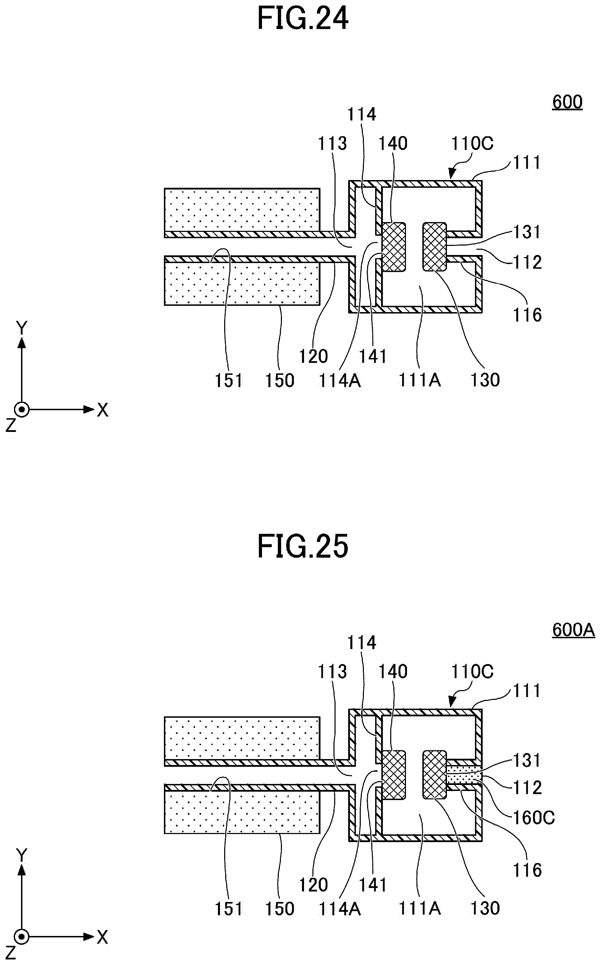

FIG. 24 is a diagram illustrating an earplug 600 according to a fifth embodiment. The earplug 600 according to the fifth embodiment has the same configuration as the earplug 100 according to the first embodiment except for a housing 110C. In the present embodiment, the housing 110C includes a main body 111, openings 112 and 113, a partition wall 114, and a pipe 116.

The pipe 116 is provided in the storage portion 111A. One end of the pipe 116 is connected to the opening 112 and extends in the -X direction side (the external auditory meatus 10 side). The microphone 130 is connected to the other end of the pipe 116 via the diaphragm 131.

By arranging the microphone 130 in the storage portion 111A via the pipe 116 as described above, any direct wind can be suppressed from hitting the microphone 130. Therefore, the level of the sound of wind collected by the microphone 130 can be reduced.

FIG. 25 is a diagram illustrating an earplug 600A according to a modification of the fifth embodiment. The earplug 600A according to the present modification is filled with an acoustic resistor 160C inside the pipe 116. The acoustic resistor 160C may be provided in a part of the inside of the pipe 116. The acoustic resistor 160C is made of the same material as the acoustic resistor 160 illustrated in the second modification of the second embodiment. The earplug 600A has the same configuration as the earplug 600 according to the fifth embodiment except that the pipe 116 is filled with the acoustic resistor 160C.

By filling the pipe 116 with the acoustic resistor 160C as described above, the inside of the pipe 116 and the microphone 130 can be protected (waterproof and dustproof). Although an acoustic signal propagating from the outside of the housing 110C to the microphone 130 is attenuated by the acoustic resistor 160C, the signal processing by the signal processing device may determine the volume level in consideration of the attenuation amount of the acoustic signal by the acoustic resistor 160C.

### <Sixth Embodiment>

Next, as in the fifth embodiment, an earplug capable of reducing the level of the sound of wind collected by the microphone 130 will be described.

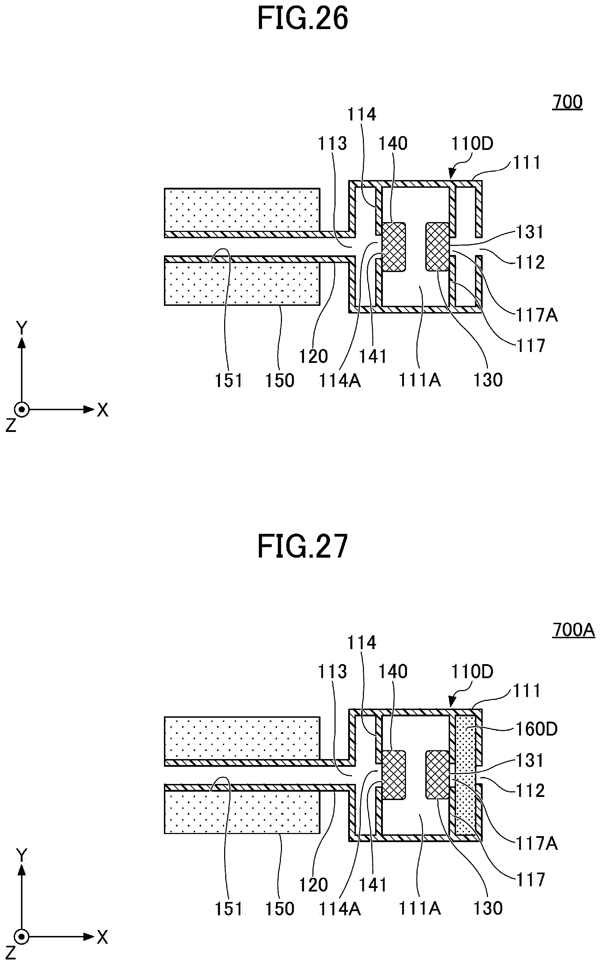

FIG. 26 is a diagram illustrating an earplug 700 according to a sixth embodiment. The earplug 700 according to the sixth embodiment has the same configuration as the earplug 100 according to the first embodiment except for a housing 110D. In this embodiment, the housing 110D includes a main body 111, openings 112, 113, and partition walls 114, 117.

The partition wall 117 is provided on the +X direction side (the opening 112 side) relative to the partition wall 114 so as to divide the storage portion 111A in the X direction. The partition wall 117 includes an opening 117A at a position facing the opening 112. The microphone 130 is connected to the partition wall 117 on the -X direction side (the external auditory meatus 10 side) of the opening 117A via the diaphragm 131.

By arranging the microphone 130 in the storage portion 111A via the partition wall 117 as described above, any direct wind can be suppressed from hitting the microphone 130, so that the level of the wind sound collected by the microphone 130 is reduced.

FIG. 27 is a diagram illustrating an earplug 700A according to a modification of the sixth embodiment. In the earplug 700A according to the present modification, the acoustic resistor 160D is filled in the area on the +X direction side (the opening 112 side) relative to the partition wall 117 in the housing 110D. The acoustic resistor 160D may be provided in a part of the area. The acoustic resistor 160D is made of the same material as the acoustic resistor 160 illustrated in the second modification of the second embodiment. The earplug 600A has the same configuration as the earplug 700 according to the sixth embodiment, except that the labor commission instrument on the +X direction side relative to the partition wall 117 in the housing 110D is filled with the acoustic resistor 160D.

By providing the acoustic resistor 160D as described above, the inside of the housing 110D and the microphone 130 can be protected (waterproof and dustproof).

In the fifth embodiment, the sixth embodiment, and each of the modifications, the sound insulation portion can be deformed in the same manner as in the first to third modifications of the second embodiment illustrated in FIG. 13 to FIG. 15.

Each of the previously described embodiments and modifications can be combined as long as contradiction does not occur.

Although the earplug, the sound collector, and the volume measuring system of the exemplary embodiments of the present invention have been described above, the present invention is not limited to the specifically disclosed embodiments, and various modifications and changes can be made without departing from the scope of claims.

This patent application claims its priority based on Japanese Patent Application No. 2019-058326 filed on March 26, 2019, and Japanese Patent Application No. 2019-141546 filed on July 31, 2019. The entire contents of Japanese Patent Application No. 2019-058326 and Japanese Patent Application No. 2019-141546 are incorporated herein by reference.

### [Reference Signs List]

- 100, 300, 400, 500, 600, 700: earplug
- 100A: sound collector
- 110: housing
- 112: opening (second opening)
- 113: opening (first opening)
- 114, 117: partition wall
- 116: pipe
- 120: tube (tubular member)
- 130: microphone (second acoustic sensor)
- 140: microphone (first acoustic sensor)
- 141: diaphragm
- 150: sound insulation portion (sound insulation member)
- 151: through hole
- 151A: non-through hole
- 152: end surface
- 160: acoustic resistor
- 170: vibration reduction member
- 180: cover
- 181: hole
- 200: volume measurement system
- 210: signal processing device (volume measuring device)

## Claims

1. An earplug comprising:
a tubular member configured to extend in one direction and to be capable of bending relative to the one direction;
a first acoustic sensor configured to collect sound via the tubular member; and
an earplug type sound insulation member in which the tubular member is inserted.

2. The earplug according to claim 1, wherein the tubular member is bendable according to a shape of an external auditory meatus.

3. The earplug according to claim 1 or claim 2 further comprising a housing having a first opening being communicated with the tubular member, and
wherein the first acoustic sensor is provided in the housing and collects sound via the tubular member and the first opening.

4. The earplug according to claim 3, wherein the tubular member and the first acoustic sensor are attached to the housing in a state of being apart from each other without direct contact.

5. The earplug according to claim 3, wherein the housing is harder than the tubular member.

6. The earplug according to claim 3 further comprising:
a second opening included in the housing, and
a second acoustic sensor provided in the housing and configured to collect sound via the second opening.

7. The earplug according to claim 6, wherein the first acoustic sensor is an acoustic sensor for collecting sound inside an external auditory meatus of a wearer, and the second acoustic sensor is an acoustic sensor for collecting sound outside of the wearer.

8. The earplug according to claim 1, wherein the sound insulation member is provided with an acoustic resistor to cover a tip of the tubular member.

9. The earplug according to claim 1 or claim 2, wherein the first acoustic sensor is arranged inside the tubular member.

10. The earplug according to claim 9, wherein a vibration reducing member is disposed in the tubular member on a side downstream to a position of the first acoustic sensor.

11. The earplug according to claim 9, wherein an acoustic resistor is disposed in the tubular member on a side upstream to a position of the first acoustic sensor.

12. A sound collector comprising:
a tubular member configured to extend in one direction and to be capable of bending relative to the one direction; and
a first acoustic sensor configured to collect sound via the tubular member.

13. A volume measurement system comprising:
an earplug; and
a volume measurement device,
wherein the earplug includes
a housing having a first opening and a second opening;
a tubular member configured to communicate with the first opening to extend and configured to be capable of bending relative to an extension direction;
a first acoustic sensor provided in the housing and collecting sound via the tubular member and the first opening;
a second acoustic sensor provided in the housing and collecting sound via the second opening; and
an earplug type sound insulation member in which the tubular member is inserted, and
wherein the volume measurement device is connected to output terminals of the first acoustic sensor and the second acoustic sensor.

14. The volume measurement system according to claim 13, wherein the volume measurement device determines a wearing state of the earplug based on a volume level difference between the first acoustic sensor and the second acoustic sensor.

15. The volume measurement system according to claim 14 further comprising a wearing state notification device configured to provide notification of the wearing state based on a determination result of the wearing state by the volume measurement device.
